(19) Europäisches Patentamt
European Patent Office
Office européen des brevets

(11) **EP 2 027 814 B1**

(12) **EUROPEAN PATENT SPECIFICATION**

(45) Date of publication and mention
of the grant of the patent:
**14.12.2011 Bulletin 2011/50**

(51) Int Cl.:
***A61B 5/00*** *(2006.01)*

(21) Application number: **08014864.6**

(22) Date of filing: **21.08.2008**

(54) **Biological observation apparatus and method for obtaining information indicative of internal state of an object using sound wave and light**

Biologische Betrachtungsvorrichtung und Verfahren zum Erhalt von Informationen, die den inneren Zustand eines Objekts mithilfe von Schallwellen und Licht anzeigen

Appareil d'observation biologique et procédé pour obtenir des informations indicatives de l'état interne d'un objet utilisant une onde acoustique et lumière

(84) Designated Contracting States:
**DE FR GB**

(30) Priority: **23.08.2007 JP 2007217488**

(43) Date of publication of application:
**25.02.2009 Bulletin 2009/09**

(73) Proprietor: **Olympus Medical Systems Corporation**
**Shibuya-ku**
**Tokyo 151-0072 (JP)**

(72) Inventor: **Igarashi, Makoto**
**Tokyo 151-0072 (JP)**

(74) Representative: **von Hellfeld, Axel**
**Wuesthoff & Wuesthoff**
**Patent- und Rechtsanwälte**
**Schweigerstrasse 2**
**81541 München (DE)**

(56) References cited:
WO-A-98/50781          DE-A1- 4 419 900
US-A1- 2005 256 403     US-B1- 6 390 978

• CHINN S R ET AL: "OPTICAL COHERENCE TOMOGRAPHY USING A FREEQUENCY-TUNABLE OPTICAL SOURCE" OPTICS LETTERS, OSA, OPTICAL SOCIETY OF AMERICA, WASHINGTON, DC, US, vol. 22, no. 5, 1 March 1997 (1997-03-01), pages 340-342, XP000684683 ISSN: 0146-9592

**Description**

Background of the Invention

(The Field of the Invention)

The present invention relates to a biological observation apparatus and a biological observation method and, more particularly, to a biological observation apparatus and a biological observation method which use a sound wave and light to optically collect information indicative of internal states of an object to be examined and observe the object.

(Related Art)

In recent years, as optical tomographic imaging, there has been known optical coherence tomography (hereinafter, referred to as "OCT").

OCT, which mainly uses light interference, obtains information of internal states of a living body. OCT enables to obtain information of the surface area of the living body in short time. This obtained information is called a tomogram. The OCT has already been put into practice in diagnosing retinopathy in an ophthalmic field, and has attracted very keen interest in the medical world.

Of technologies of the above OCT, one technology related to the spectral-swept OCT is described in "T. Yoshimura and O. Matoba, "Biological Tomography Measurement Using Spectral-swept Interferometry" OPTICAL AND ELECTRO-OPTICAL ENGNEERING CONTACT, Vol.41, No.7, pp.418-425, 2003" (non-patent reference (1)). According to the spectral-swept OCT, biological information of plural portions of a living body positioned in the depth direction can be obtained by changing in order the wavelength of light emitted from a light source.

In addition, "M. Hisaka, T. Sugiura, and S. Kawata, "Optical Tomography of a Thick Scattering Medium Using Interaction between Pulsed Ultrasound and Light" KOGAKU 29, pp631-634, 2000" (non-patent reference (2)) discloses a technology in which an ultrasound wave and light are radiated into a living body, and the light modulated in the living body by the pulsed ultrasound wave is detected to obtain an optical image of a portion positioned about 1 cm lower than the superficial layer of the mucosa.

However, the spectral-swept OCT described in the non-patent reference (1) has a problem that S/N of the biological information falls when it is obtained from a portion positioned a few millimeters (mm) or more lower than the surface of the living body. This is because there is a case where object light, which is generated when the light emitted from a light source is modulated in the living body, and reference light, which is substantially identical with the light emitted from the light source, do not properly interfere with each other.

The optical imaging described in the non-patent reference (2) has a problem that since point scanning is used, a very long time is required for obtaining biological information of a desired area of the living body.

DE 44 19 900 discloses a method in which examination light and focused ultrasonic radiation are beamed into an object. After passing through the object, the examination light is superimposed in an interferometer on reference light which is coherent with the examination light. Intensity or amplitude of resulting interference light provides information for an image point which corresponds to an image of a part of the object lying in a zone of focus of the focussed ultrasonic radiation.

US 2005/256403 discloses an imaging apparatus including a source of electromagnetic radiation that is configured to emit electromagnetic radiation at two or more wavelengths through an imaging volume. The imaging apparatus also includes an ultrasound transducer that is configured to direct ultrasound waves through the imaging volume such that electromagnetic radiation passing through the imaging volume is modulated at a frequency of the ultrasound waves. The imaging apparatus further includes one or more electromagnetic radiation detectors that are configured to detect modulated electromagnetic radiation from the imaging volume.

WO 98/50781 discloses an apparatus for frequency encoded ultrasound modulated optical tomography. A function generator produces a frequency sweep signal which is applied to an ultrasonic transducer. The ultrasonic transducer produces an ultrasonic wave in a turbid medium. Coherent light from a laser is passed through turbid medium where it is modulated by the ultrasonic wave. A photomultiplier tube detects light which passes through the turbid medium. The signal from the photomultiplier tube is fed to an oscilloscope and then to a computer where differences in light intensity at different frequencies can determine a location of objects in the turbid medium.

Summary of the Invention

The present invention has been made in consideration of the foregoing conventional situation, and an object of the present invention is to provide biological observation apparatus and method which obtains information of a deeper portion of an object to be examined with high S/N and quickly.

In order to achieve the above object, a biological observation apparatus according to the present invention comprises: a sound wave radiating unit that radiates a sound wave into an object to be examined, the sound wave having a predetermined wavelength or frequency; a control unit that controls a radiation state of the sound wave radiated from the sound wave radiating unit to the object; a light radiating unit that radiates light into the object while successively changing a wavelength of the light; a light receiving unit that receives object light generated when the light radiated from the light radiating unit is reflected at an area inside the object; a light detector that obtains first interference light caused by interference between the radiated light and first object light received by the light receiving unit when the sound wave is controlled not to be radiated by the control unit, and obtains second interference light caused by interference between the radiated light and second object light received by the light receiving unit when the sound wave is controlled to be radiated by the control unit; and a calculation unit that calculates information of an area inside the object where the sound wave is radiated, based on the first Interference light and the second interference light obtained by the light detector, the information being indicative of a scattered state of the radiated light.

Brief Description of the drawings

In the accompanying drawings:

Fig. 1 is a block diagram exemplifying an outline of a biological observation apparatus according to an embodiment of the present invention;
Fig. 2 is a flowchart showing operations of the biological observation apparatus;
Fig. 3 is a diagram showing an interrelation between variations with time of frequencies of object light and reference light detected from a first interference signal and values calculated based on the first interference signal;
Fig. 4 is a diagram showing an interrelation between variations with time of frequencies of object light and reference light detected from a second interference signal and values calculated based on the second interference signal;
Fig. 5 is a block diagram showing a modification example of the biological observation apparatus according to the embodiment of the present invention;
Fig. 6 is a diagram showing a configuration of an optical coupler of the modification example in detail; and
Fig. 7 is a sectional diagram exemplifying a configuration of an end part of an optical fiber of the modification example.

Detailed Description of the Preferred Embodiments

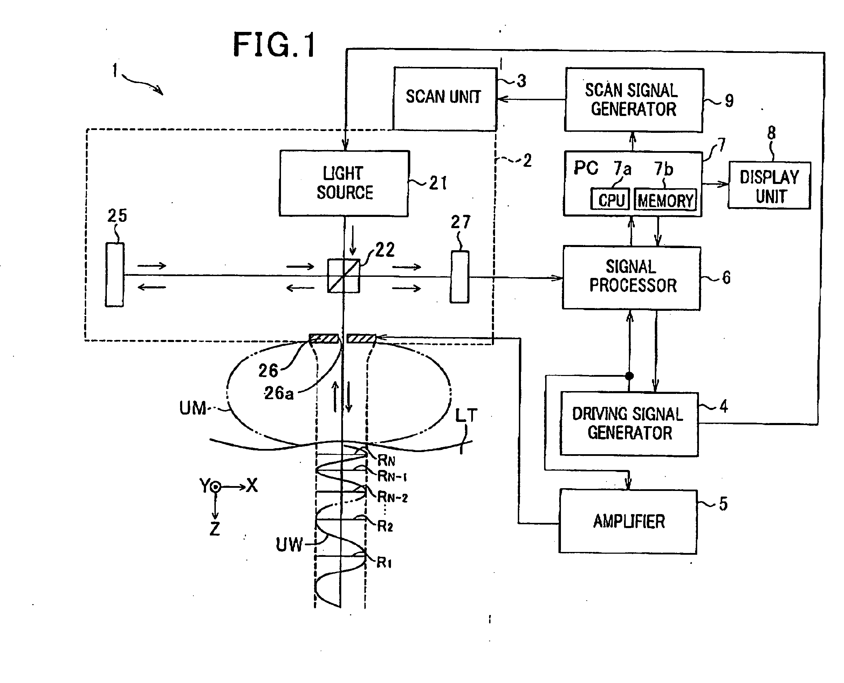

Hereinafter, preferred embodiments of the present invention will now be described in connection with the accompanying drawings.
Referring to Figs. 1 - 7, a biological observation apparatus according to an embodiment of the present invention will now be described.
Fig. 1 outlines a biological observation apparatus 1. This biological observation apparatus 1 comprises, as shown in Fig. 1, a radiation/reception unit 2 which radiates an ultrasound wave and light toward a living tissue (body tissue) LT serving as an object to be examined and receives light produced by the reflection and scattering of the radiated light from the living tissue LT (hereinafter, the reflected and scattered light is referred to as "object light"). Moreover, the biological observation apparatus 1 comprises a scan unit 3, a driving signal generator 4, an amplifier 5, a signal processor 6, a personal computer (hereinafter, called PC) 7, a display unit 8, and a scan signal generator 9.
The scan unit 3 is configured to, in response to a scan signal issued from the scan signal generator 9, change the spatial position of the radiation/reception unit 2 (i.e., scan position) with respect to the living tissue LT and radiate an ultrasound wave and light.
The driving signal generator 4 produces a light source drive signal to make the radiation/reception unit 2 radiate the light whose frequencies successively change, and outputs the produced light source drive signal. The driving signal generator 4 produces an ultrasound wave drive signal to make the radiation/reception unit 2 radiate the ultrasound wave having a predetermined wavelength (or a predetermined frequency), and outputs the produced ultrasound wave drive signal. In addition, the driving signal generator 4 serving as a control unit changes the output state of the light source drive signal and the ultrasound wave drive signal under the control of the signal processor 6. Specifically, the driving signal generator 4 changes the output state of the ultrasound wave drive signal under the control of the signal processor 6 to switch the radiation state of the ultrasound wave, which is radiated from (the ultrasound transducer 26 of) the radiation/reception unit 2, from "off" to "on" or vice versa.
The amplifier 5 includes a power amplifier. This amplifier 5 amplifies the power of an ultrasound wave drive signal outputted from the driving signal generator 4, and provides the amplified ultrasound wave drive signal to the radiation/reception unit 2.
The radiation/reception unit 2, serving as a photo detector, is provided with a light source 21, a half mirror 22, a reference mirror 25, an ultrasound transducer 26 with an opening 26a formed at its center, and a light detector 27.

EP 2 027 814 B1

The light source 21 emits light which can enter a living tissue LT, during which the light source 21 successively changes the wavelength of the light. The light source 21 is composed of, for example, a wavelength tunable laser source (not shown) or a combination of a SLD (Super Luminescent Diode) and an interference filter (not shown). The light source 21 emits light to the half mirror 22 while successively changing the wavelength of the light emitted therefrom, for example, from the maximum wavelength $\lambda_{max}$ to the minimum wavelength $\lambda_{min}$ based on the light source drive signal provided from the driving signal generator 4. In the present embodiment, the light emitted from the light source 21 may be continuous light or pulsed light. In addition, the light source 21 is configured to emit light having a center wavelength, for example, in a range from 600 nm to 1000 nm. However, the configuration of the light source 21 is not limited to the above configuration.

Of the light which has traveled from the light source 21, the half mirror 22 reflects part of that light so that the reflected light is radiated toward the reference mirror 25 and allows the remaining part of that light to be transmitted toward the ultrasound transducer 26. The light which has traveled from the half mirror 22 to the reference mirror 25 is reflected by the reference mirror 25, and then made to enter the half mirror 22 as reference light. The light which has traveled from the half mirror 22 to the ultrasound transducer 26 passes through the opening 26a, before being radiated toward the living tissue LT.

In the present embodiment, a space between (the ultrasound transducer 26 of) the radiation/reception unit 2 and the living tissue LT is filled with an ultrasound transmissive medium UM, which is, for example, water, before each unit of the biological observation apparatus 1 performs operations for obtaining biological information of the living tissue LT.

The ultrasound transducer 26 functions as an ultrasound wave generator. This ultrasound transducer 26 is driven in response to the ultrasound wave drive signal provided from the driving signal generator 4 and radiates a predetermined ultrasound wave, which is a continuous wave, toward the living tissue LT along the axis of light passing the opening 26a. The ultrasound wave has a frequency of several megahertzs (MHz) to several tens of megahertzs (MHz). This ultrasound wave travels through the inside of the living tissue LT as a cyclic compressional wave. In the present embodiment, the ultrasound wave is radiated toward the living tissue LT without being converged. But, a convergence means such as an acoustic lens may be used to converge the ultrasound wave so that the converged ultrasound wave is radiated to the living tissue LT. Note that the ultrasound wave radiated from the ultrasound transducer 26 is not limited to the continuous wave. For example, a pulse wave may be used.

While the ultrasound transducer 26 does not radiate the ultrasound wave (that is, the radiation state of the ultrasound wave is "off"), the light radiated from the radiation/reception unit 2 travels inside the living tissue LT in the depth direction until reaching a portion (area) associated with the wavelength of the light. The light is reflected at the portion. The reflected light passes through the opening 26a, then entering the half mirror 22 as first object light.

On the other hand, while the ultrasound transducer 26 radiates the ultrasound wave (that is, the radiation state of the ultrasound wave is "on"), the light radiated from the radiation/reception unit 2 is reflected at a portion (area) whose density is maximized by the ultrasound wave. The reflected light passes through the opening 26a, then entering the half mirror 22 as second object light. That is, the light transmitted through the half mirror 22 is reflected at the portion inside the living tissue LT whose density is increased by the ultrasound wave, then entering the half mirror 22 as the object light.

The half mirror 22 functions as a light splitting unit for splitting light in two. The half mirror 22 allows the reference light coming from the reference mirror 25 to interfere with the object light coming from the ultrasound transducer 26, so that interference light, which is caused by the interference between the two fluxes of light, is radiated toward the light detector 27.

The light detector 27 serving as an interference signal output unit applies heterodyne detection to the interference light coming from the half mirror 22, and converts the detected interference light into an interference signal, which is an electric signal, to output the interference signal to the signal processor 6.

The signal processor 6 is provided with, for example, a spectrum analyzer or a digital oscilloscope and functions as a calculation unit. The signal processor 6 calculates a beat frequency $f_b$ associated with the timing when the wavelength of the light emitted from the light source 21 is changed; based on a first interference signal and a light source drive signal. The first interference signal is inputted at the timing when light, whose wavelength is successively changed, is emitted to the living tissue LT. The light source drive signal is outputted from the driving signal generator 4. The signal processor 6 outputs the result of the calculation to the PC 7. The calculation of the beat frequency $f_b$ will be described in detail later.

Furthermore, after the calculation of the beat frequency $f_b$ associated with the timing when the wavelength of the light emitted from the light source 21 is successively changed is completed, the signal processor 6 controls the driving signal generator 4 to radiate a predetermined ultrasound wave from the ultrasound transducer 26 to the living tissue LT and to successively change the frequency of the light emitted from the light source 21 from the maximum wavelength $\lambda_{max}$ to the minimum wavelength $\lambda_{min}$. Then, the signal processor 6 calculates the sum of $f_b$ and $f_d$ (i.e., $f_b + f_d$), where $f_b$ is a beat frequency associated with the timing when the wavelength of the light emitted from the light source 21 is changed and $f_d$ is Doppler shift amount (the amount of frequency modulation) associated with the above timing, based on a second interference signal inputted under the above control and both the ultrasound wave drive signal and the light source drive signal outputted from the driving signal generator 4. The signal processor 6 outputs the result of the calculation to the PC 7. The calculation of $(f_b + f_d)$ will be described in detail later.

The PC 7 comprises a CPU (central processing unit) 7a, which performs various types of calculation and processing, and a memory 7b serving as a storage unit and stores various data and others. The CPU 7a of the PC 7, which functions as a calculation unit, performs calculation based on the beat frequency $f_b$ and the value of $(f_b + f_d)$. Thus, image data of the living tissue LT in the depth direction (Z-axis direction shown in Fig. 1) are produced. In addition, the PC 7 relates the produced image data to scan positional information which shows positions within a scan range (X-axis and Y-axis directions) where the scan can be performed by the scan unit 3, and the image data and the scan positional information, which are related to each other, are stored in the memory 7b.

Moreover, the CPU 7a of the PC 7 determines whether or not the current scan position that gives the image data is an end position of the scan range controlled by the scan unit 3. When it is determined that the current scan position is not the end, the scan signal generator 9 is controlled to change the scan position and radiate the ultrasound wave and light. The CPU 7a of the PC 7 also determines whether or not the image data for one frame have been stored in the memory 7b. When the determination for this storage is affirmative, the image data are converted to image signals to be outputted to the display unit 8.

The scan signal generator 9 provides scan signals to the scan unit 3 under the control of the PC 7 to radiate the ultrasound wave and light while changing the scan position in the X-axis and Y-axis directions.

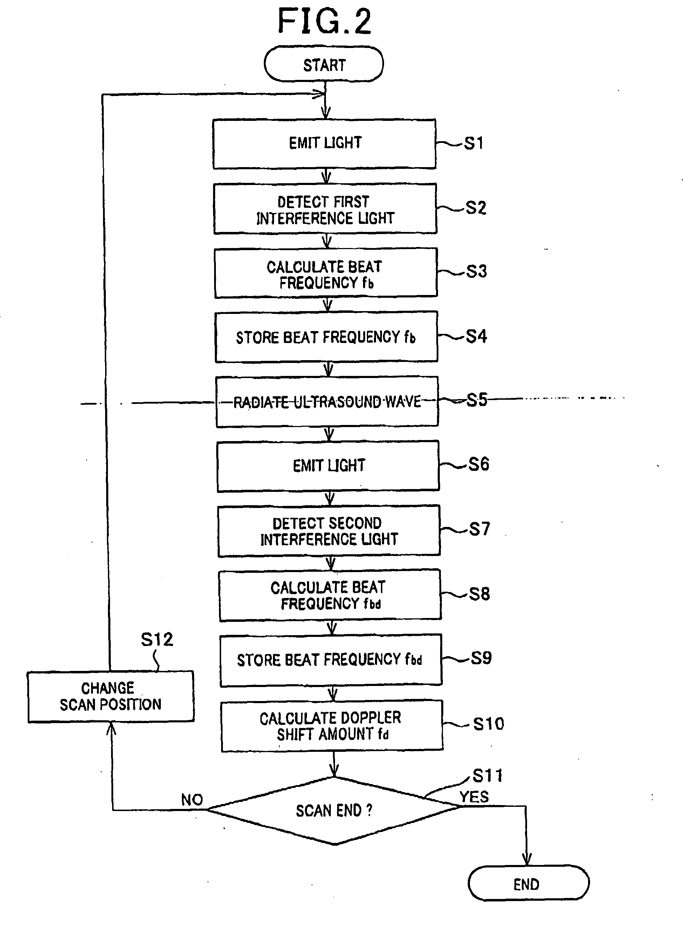

The operations of the biological observation apparatus 1 according to the present embodiment will now be described with reference to a flowchart shown in Fig. 2.

First, an operator powers each part of the biological observation apparatus 1, and positions the ultrasound transducer 26 of the radiation/reception unit 2 such that the ultrasound wave and light are radiated in the Z-axis direction shown in Fig. 1 (i.e., the depth direction of the living tissue LT). Concurrently, a space between the ultrasound transducer 26 and the living tissue LT is filled with the ultrasound transmissive medium UM, for example, water.

The operator then turns on switches, which are mounted in an operation device (not shown), to instruct to start obtaining biological information of the living tissue LT.

The driving signal generator 4 generates in response to this instruction a light source drive signal for radiating light, whose wavelength is successively changed (decreased) from the maximum wavelength $\lambda_{max}$ to the minimum wavelength $\lambda_{min}$, from the radiation/reception unit 2 and provides the light source drive signal to the light source 21.

In step S1, the light source 21 emits light to the half mirror 22 while the light source 21 successively changes (decreases) the wavelength of the light emitted therefrom from the maximum wavelength $\lambda_{max}$ to the minimum wavelength $\lambda_{min}$, based on the light source drive signal provided from the driving signal generator 4.

The part of the light emitted from the light source 21 passes through the half mirror 22 and is radiated through the opening 26a in the Z-axis direction (the depth direction in the living tissue LT). The light radiated through the opening 26a travels inside the living tissue LT in the depth direction until reaching a portion associated with the wavelength of the light. The light is reflected at the portion. The reflected light passes through the opening 26a, then entering the half mirror 22 as first object light.

On the other hand, another part of the light emitted from the light source 21 is reflected by the half mirror 22 and is radiated to the reference mirror 25. The light radiated to the reference mirror 25 is reflected by the reference mirror 25, and then made to enter the half mirror 22 as reference light.

The half mirror 22 allows the reference light coming from the reference mirror 25 to interfere with the first object light coming from the ultrasound transducer 26, so that interference light, which is caused by the interference between the two fluxes of light, is radiated toward the light detector 27 as first interference light.

In step S2, the light detector 27 applies heterodyne detection to the first interference light coming from the half mirror 22, and converts the detected first interference light into a first interference signal, which is an electric signal, to output the first interference signal to the signal processor 6. In step S3, the signal processor 6 calculates a beat frequency $f_b$ associated with the timing when the wavelength of the light emitted from the light source 21 is changed, based on the first interference signal and the light source drive signal provided from the driving signal generator 4.

Hereinafter, the calculation of the beat frequency $f_b$ performed by the signal processor 6 will be described.

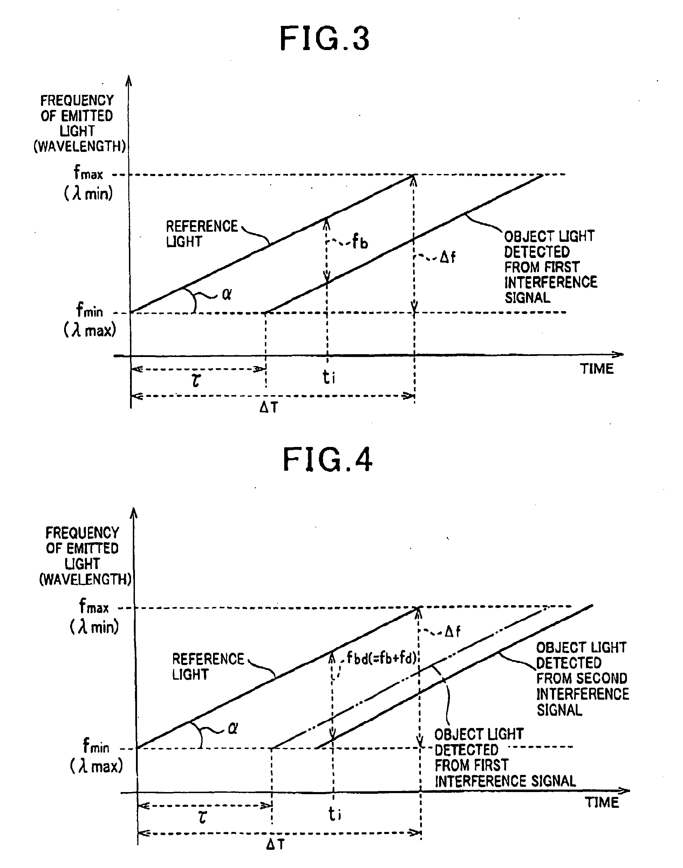

The frequency of the light emitted from the light source 21 is successively increased from the minimum frequency $f_{min}$ to the maximum frequency $f_{max}$, as the wavelength of the light successively decreases from the maximum wavelength $\lambda_{max}$ to the minimum wavelength $\lambda_{min}$. That is, as the frequency of the light is successively increased, the frequencies of the reference light and the object light, which interfere with each other, are also successively increased. Note that, in the present embodiment, both of the frequency of the reference light and the frequency of the object light linearly increase as shown in Fig. 3. Fig. 3 shows an interrelation between variations with time of the frequencies of the object light and the reference light detected from the first interference signal and values calculated based on the first interference signal.

In the present embodiment, while the wavelength of the light emitted from the light source 21 is successively changed from the maximum wavelength $\lambda_{max}$ to the minimum wavelength $\lambda_{min}$, firstly the light having the maximum

wavelength $\lambda_{max}$ is radiated from the radiation/reception unit 2, and lastly the light having the minimum wavelength $\lambda_{min}$ is radiated the radiation/reception unit 2. In a case where the radiated light is reflected at the deep layer (the deepest layer subjected to the radiation) of the living tissue LT, the light enters the half mirror 22 as object light having the longest delay time with respect to the reference light. In a case where the radiated light is reflected at the surface layer (the shallowest layer subjected to the radiation) of the living tissue LT, the light enters the half mirror 22 as object light having the shortest delay time with respect to the reference light.

The frequency change rate $\alpha$ which indicates the amount of variations in the frequency of the light per unit time is calculated by the following equation (1):

$$\alpha = \Delta f / \Delta T \qquad \cdots (1)$$

where $\Delta f$ is the frequency change range ($f_{max} - f_{min}$) of the light radiated from the radiation/reception unit 2, and $\Delta T$ is the frequency change time which is the time required for changing the frequency of the light from the minimum frequency $f_{min}$ to the maximum frequency $f_{max}$.

The delay time $\tau$, which indicates a period of time between the time when the reference light enters the half mirror 22 and the time when the object light enters the half mirror 22, is calculated by the following equation (2):

$$\tau = 2nz / c \qquad \cdots (2)$$

where c is the speed of light, n is the index of refraction of the living tissue LT, and z is the depth of one portion positioned in the depth direction (Z-axial direction).

Then, the beat frequency $f_b$ associated with one timing $t_i$ within the frequency change time $\Delta T$ ($0 < t_i \leq \Delta T$) is calculated by the following equation (3):

$$f_b = \alpha \times \tau = (\Delta f / \Delta T) \times (2nz / c) \qquad \cdots (3)$$

That is, the signal processor 6 uses the above equations (1) to (3) based on the first interference signal and the light source drive signal provided from the driving signal generator 4 to calculate the beat frequency $f_b$ associated with respective delay time $\tau$ and provides the result of the calculation to the PC 7.

Note that Fig. 3 shows the interrelation between variations with time of the frequencies of the object light and the reference light detected from the first interference signal and values of the frequency change range $\Delta f$ of the light, the frequency change time $\Delta T$, the frequency change rate $\alpha$, the delay time $\tau$, and the beat frequency $f_b$, which are parameters used in the above equations (1) to (3).

After the calculation of the beat frequency $f_b$ based on respective delay time $\tau$ is completed, the signal processor 6 controls the driving signal generator 4 to radiate a predetermined ultrasound wave from the ultrasound transducer 26 to the living tissue LT and to successively change the frequency of the light emitted from the light source 21 from the maximum wavelength $\lambda_{max}$ to the minimum wavelength $\lambda_{min}$.

Meanwhile, in step S4, the PC 7 stores the values of the beat frequency $f_b$ outputted from the signal processor 6 in the memory 7b in order.

The driving signal generator 4 produces a light source drive signal to make the radiation/reception unit 2 radiate the light whose wavelength successively changes from the maximum wavelength $\lambda_{max}$ to the minimum wavelength $\lambda_{min}$ and outputs the light source drive signal to the light source 21 under the control of the signal processor 6. In addition, the driving signal generator 4 produces an ultrasound wave drive signal to make the radiation/reception unit 2 radiate the ultrasound wave having a predetermined wavelength (or frequency) and outputs the ultrasound wave drive signal to the ultrasound transducer 26 via the amplifier 5 under the control of the signal processor 6.

In step S5, the ultrasound transducer 26 radiates an ultrasound wave, which is a continuous wave, toward the living tissue LT along the axis of light passing the opening 26a in response to the ultrasound wave drive signal provided from the driving signal generator 4. Then, the ultrasound wave radiated from the ultrasound transducer 26 travels inside the living tissue LT as a periodic compressional wave. That is, the ultrasound wave radiated from the ultrasound transducer 26 travels inside the living tissue LT in its depth direction as a longitudinal compressional wave from its lower-frequency wave parts (i.e., longer wavelengths) in sequence. Hence, the frequencies of the ultrasound wave entering the living tissue become higher over time (i.e., the wavelengths become shorter). Note that the ultrasound wave radiated from the ultrasound transducer 26 may be pulsed light, and not limited to the continuous light.

The ultrasound wave traveling inside the living tissue LT applies pressure to the internal tissue depending on its degrees of compression determined by its intensity (i.e., amplitude), which will give local compression to the tissue so that the tissue density is locally changed. This results in that, as indicated by UW in Fig. 1, the densities of the living tissue LT are locally maximized (i.e., made higher) at each of Z-axis (depth directional) directional positions (portions) in the living tissue LT, where such positions spatially correspond to the maximum intensities of the ultrasound wave which is transmitted inside the living tissue LT.

Those locally compressed tissue portions are greater in density than the other portions, so that such locally density-maximized tissue portions are able to strongly reflect (and scatter) light. In Fig. 1, these local density-maximized tissue portions positioned in the Z-axis direction inside the living tissue LT are shown as wave fronts $R_1$, $R_2$, $\cdots$, $R_N$ of the ultrasound wave (hereinafter referred to as "ultrasound wave fronts"). At a time instant when the radiation of the ultrasound wave has just been completed, the ultrasound wave fronts $R_1$, $R_2$, $\cdots$, $R_N$ are spatially located along the ultrasound transmission direction (the Z-axis direction) in sequence.

Meanwhile, in step S6, the light source 21 emits light to the half mirror 22 while the light source 21 successively changes (decreases) the wavelength of the light emitted therefrom from the maximum wavelength $\lambda_{max}$ to the minimum wavelength $\lambda_{min}$, based on the light source drive signal provided from the driving signal generator 4.

A part of the light radiated from the light source 21 passes through the half mirror 22 and is then radiated through the opening 26a in the Z-axis direction (the depth direction in the living tissue LT) shown in Fig. 1. In the living tissue LT, the light radiated through the opening 26a encounters each of the plurality of ultrasound wave fronts $R_1$, $R_2$, $\cdots$, $R_N$, resulting in that the light is partly reflected (including scattering) by each wave front. The reflected light, which has come from each of the ultrasound wave fronts $R_1$, $R_2$, $\cdots$, $R_N$, is returned to the half mirror 22 via the opening 26a and a modulator (not shown) as object light (reflected light) that has been subjected to Doppler shift (i.e., frequency modulation) with a frequency $f_d$.

On the other hand, another part of the light emitted from the light source 21 is reflected by the half mirror 22 and is radiated to the reference mirror 25. The light radiated to the reference mirror 25 is reflected by the reference mirror 25, and then made to enter the half mirror 22 as reference light.

The half mirror 22 allows the reference light coming from the reference mirror 25 to interfere with the object light coming from the ultrasound transducer 26, so that interference light, which is caused by the interference between the two fluxes of light, is radiated toward the light detector 27 as second interference light.

In step S7, the light detector 27 applies heterodyne detection to the second interference light coming from the half mirror 22, and converts the detected second interference light into a second interference signal, which is an electric signal, to output the second interference signal to the signal processor 6. In step S8, the signal processor 6 calculates a beat frequency $f_{bd}$ associated with the timing when the wavelength of the light emitted from the light source 21 is changed, based on the second interference signal and both the ultrasound wave drive signal and the light source drive signal provided from the driving signal generator 4.

Hereinafter, the calculation of the beat frequency $f_{bd}$ performed by the signal processor 6 will be described.

In the living tissue LT, the index of refraction of each portion at which each of the wave fronts $R_1$, $R_2$, $\cdots$, $R_N$ of the ultrasound wave is located is expressed as $(n + \Delta n)$, where $n$ is the index of refraction of the living tissue LT, and $\Delta n$ is the variation of the index of refraction generated when the density of the living tissue LT is maximized by the ultrasound wave radiated from the ultrasound transducer 26. That is, the beat frequency $f_{bd}$ is calculated by the following equation (4):

$$\begin{aligned} f_{bd} &= f_b + f_d \\ &= \alpha \times \tau + f_d \\ &= (\Delta f / \Delta T) \times (2nz / c) + 2V(n + \Delta n) / \lambda \qquad \cdots (4) \end{aligned}$$

where V is the speed of the ultrasound wave radiated from the ultrasound transducer 26, and $\lambda$ is the wavelength of the ultrasound wave. As shown above, the beat frequency $f_{bd}$ is calculated by adding $f_d$, which is Doppler shift amount associated with one timing $t_i$ within the frequency change time $\Delta T$, to the beat frequency $f_b$ associated with the timing $t_i$.

Note that the speed V of the ultrasound wave and the wavelength $\lambda$ of the ultrasound wave are (given) values detected based on the ultrasound wave drive signal provided from the driving signal generator 4.

That is, the signal processor 6 uses the above equations (1) to (4) based on the second interference signal and both the ultrasound wave drive signal and the light source drive signal provided from the driving signal generator 4 to calculate the beat frequency $f_{bd}$ associated with respective delay time $\tau$, and provides the result of the calculation to the PC 7.

Note that Fig. 4 shows the interrelation between variations with time of the frequencies of the object light and the reference light detected from the second interference signal and values of the frequency change range $\Delta f$, the

frequency change time $\Delta T$, the frequency change rate $\alpha$, the delay time $\tau$, and the beat frequency $f_{bd}$, which are parameters used in the above equations (1) to (4).

After the calculation of the beat frequency $f_{bd}$ associated with respective delay time $\tau$ is completed, the signal processor 6 controls the driving signal generator 4 to stop radiating the ultrasound wave from the ultrasound transducer 26. Note that the signal processor 6 may control the driving signal generator 4 to radiate the ultrasound wave from the ultrasound transducer 26, and to emit light from the light source 21 simultaneously with or after stopping the radiation of the ultrasound wave.

Meanwhile, in step S9, the PC 7 stores the values of the beat frequency $f_{bd}$ outputted from the signal processor 6 in the memory 7b in order. The CPU 7a of the PC 7 sorts the values of the beat frequency stored in the memory 7b, for example, in descending order, according to the time when the values are stored. Thereby, the beat frequency $f_b$ associated with one timing $t_i$ and the beat frequency $f_{bd}$ associated with the timing $t_i$ are identified.

Next, in step S10, the CPU 7a subtracts the beat frequency $f_b$ associated with one timing $t_i$ from the beat frequency $f_{bd}$ associated with the timing $t_i$ to obtain the Doppler shift amount $f_d$ associated with the timing $t_i$.

Then, the CPU 7a divides the Doppler shift amount $f_d$ by $(2V / \lambda)$, which is twice the frequency of the ultrasound wave radiated from the ultrasound transducer 26, to obtain $(n + \Delta n)$ which is scattering information (information indicative of a scattered state).

Meanwhile, the values of the beat frequency $f_b$ and the beat frequency $f_{bd}$ increase in proportion to the value of the delay time $\tau$ as shown in the equations (3) and (4). Then, the delay time of the object light reflected at the deep layer of the living tissue LT is the value which is closest to the frequency change time $\Delta T$ (the beat frequency $f_b$ and the beat frequency $f_{bd}$ are maximized), and the delay time of the object light reflected at the surface layer of the living tissue LT is the value which is closest to "0" (the beat frequency $f_b$ and the beat frequency $f_{bd}$ are minimized). That is, the CPU 7a can uniquely identify the value of $(n + \Delta n)$ (scattering information) of each portion, which is positioned between the deep layer and the surface layer and on which each of the wave fronts $R_1$, $R_2$, $\cdots$, $R_N$ of the ultrasound wave is generated as shown Fig. 1, by referring to the sorted values of the beat frequency $f_b$ and the beat frequency $f_{bd}$. In consequence, the signal processor 6 and the PC 7 can obtain at one time the biological information of portions (N in number), which are positioned in the Z-axis direction (i.e., the depth direction of the living tissue LT) and located in the observation position required by the operator.

After the value of $(n + \Delta n)$ of each portion is identified on which each of the wave fronts $R_1$, $R_2$, $\cdots$, $R_N$ of the ultrasound wave is generated as shown Fig. 1, the CPU 7a maps the values of $(n + \Delta n)$ to produce image data of portions positioned in the Z-axis direction (i.e., the depth direction of the living tissue LT). The CPU 7a relates the produced image data to scan positional information, which shows positions within a scan range (X-axis and Y-axis directions), where the scan can be performed by the scan unit 3, and stores the image data and the scan positional information, which are related to each other, in the memory 7b. In step S11, when the CPU 7a detects a state in which the current scan position that gives the image data is not the end position of the scan range controlled by the scan unit 3, in step S12, the CPU 7a controls the scan signal generator 9 to change the scan position in the X-axis or Y-axis direction shown in Fig. 1, and to radiate the ultrasound wave and light.

When the signal processor 6 detects a state in which the control for changing the scan position is performed by the PC 7, the signal processor 6 controls the driving signal generator 4 to successively change the wavelength of the light emitted from the light source 21 from the maximum wavelength $\lambda_{max}$ to the minimum wavelength $\lambda_{min}$ to obtain the first interference signal at the changed scan position. Thereafter, each part of the biological observation apparatus 1 repeatedly performs the above described operations from step S1 until the scan position reaches the end position of the scan range controlled by the scan unit 3.

Then, when the CPU 7a of the PC 7 detects a state in which the scan position of the produced image data reaches the end position of the scan range controlled by the scan unit 3, that is, a state in which image data for one frame are stored in the memory 7b, the CPU 7a converts the image data into a video signal and outputs the video signal to the display unit 8. Thereby, the display unit 8 displays a tomogram (two-dimensional image or three-dimensional image) of the observation area of the living tissue LT required by the operator.

As described above, the biological observation apparatus 1 of the present embodiment can uniquely relate the portions positioned in the depth direction, whose density is increased by the sound wave, to the scattering information of the portions, based on the first beat frequency obtained when only the light is radiated to the living tissue LT and the second beat frequency obtained when both the light and the sound wave are radiated to the living tissue LT. In consequence, the biological observation apparatus 1 of the present embodiment can obtain the information (biological information) of the deep portions of the object to be examined (living body) with higher S/N and more quickly than conventional ways.

In the above described embodiment, the directions in which the ultrasound wave and the light are radiated are parallel to the Z-axis direction shown in Fig. 1, but this is not always a definitive structure. For example, any one of the ultrasound wave and the light may be radiated at an angle to the Z-axis direction.

In addition, the state of the light emitted from the light source 21 based on the light source drive signal provided from the driving signal generator 4 is not limited to the above described state in which the wavelength of the light successively decreases from the maximum wavelength $\lambda_{max}$ to the minimum wavelength $\lambda_{min}$. The wavelength of the light may successively increase from the minimum wavelength $\lambda_{min}$ to the maximum wavelength $\lambda_{max}$. In addition, in the above described embodiment, the operation is repeatedly performed in which the first interference signal and the second interference signal are obtained at one scan position and thereafter the scan position is changed within the scan range. However, an operation may be performed in which the first interference signals are obtained at all scan positions within the scan range and thereafter the second interference signals are obtained at all the scan positions within the same scan range.

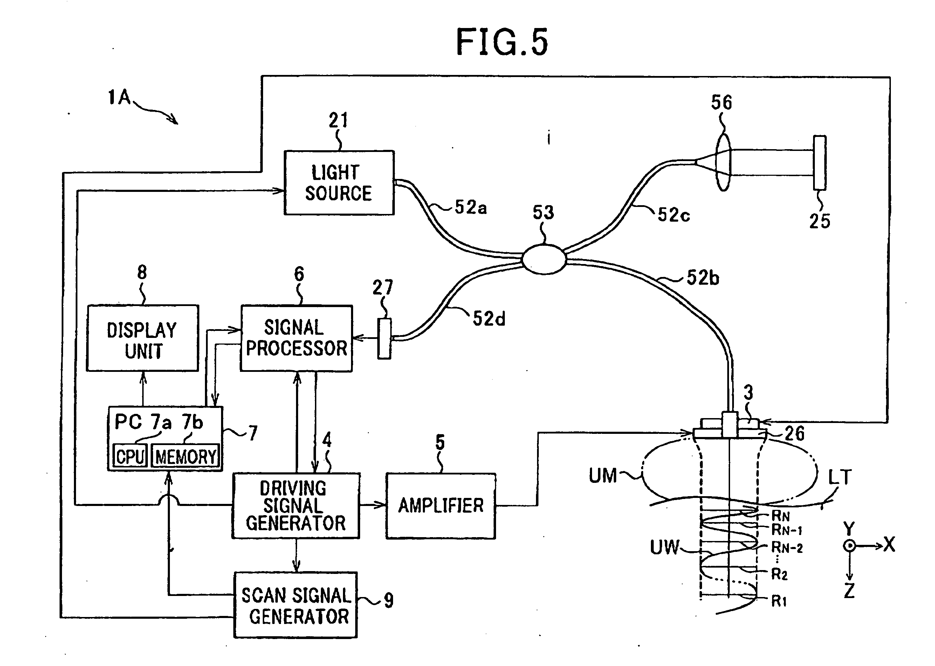

Meanwhile, to obtain advantages similar to the above, the biological observation apparatus 1 shown in Fig. 1 may be modified into a biological observation apparatus 1A shown in Fig. 5. The same reference numerals as in Fig.1 denote the same parts in Fig. 5.

Practically, the biological observation apparatus 1A comprises, as essential components, optical fibers 52a, 52b, 52c, and 52d, an optical coupler 53, and a collimating lens 56, in addition to the scan unit 3, driving signal generator 4, amplifier 5, signal processor 6, PC7, display unit 8, scan signal generator 9, light source 21, reference mirror 25, ultrasound transducer 26, and light detector 27.

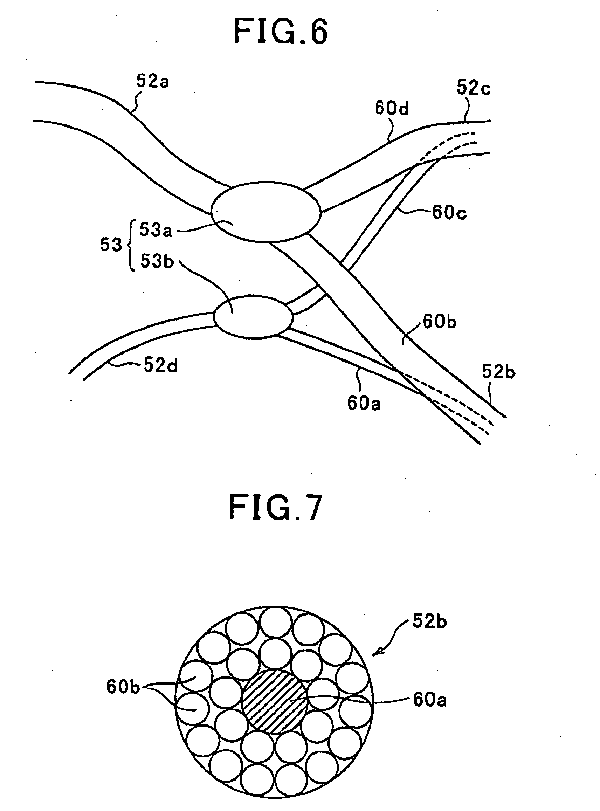

The optical coupler 53 comprises, as shown in Fig. 6, a first coupler 53a and a second coupler 53b.

The first coupler 52a has one end connected to the light source 21 and the other end connected to the first coupler 53a, as shown in Figs. 5 and 6.

The optical fiber 52b comprises, as shown in Fig. 6, a light-receiving fiber bundle 60a and a light-sending fiber bundle 60b. The fiber bundle 60a has one end connected to the second coupler 53b and the other end connected to an opening (for example, though not shown in Fig. 5, the opening 26a) formed at the center of the ultrasound transducer 26 in such a manner that the other end is inserted therethrough. Meanwhile, the fiber bundle 60b has one end connected to the first coupler 53a and the other end connected to an opening (for example, though not shown in Fig. 5, the opening 26a) formed at the center of the ultrasound transducer 26 in such a manner that the other end is inserted therethrough. Both other ends of the respective fiber bundles 60a and 60b are arranged at the opening of the ultrasound transducer 26 as illustrated in Fig. 7. In Fig. 7, the fiber bundle 60a is adapted to a core and surrounded by the fiber bundle 60b.

The optical fiber 52c also comprise, as shown in Fig. 6, a light-receiving fiber bundle 60c and a light-sending fiber bundle 60d. The fiber bundle 60c has one end connected to the second coupler 53b and the other end arranged at a predetermined position where the light comes in from the collimating lens 56. Moreover, the fiber bundle 60d has one end connected to the first coupler 53a and the other end arranged at a predetermined position where the light can be radiated to the collimating lens 56.

The optical fiber 52d has, as shown in Figs. 5 and 6, one end connected to the second coupler 53b and the other end connected to the light detector 27.

As described above, in this biological observation apparatus 1A, the light emitted from the light source 21 while the wavelength thereof is successively changed (decreased) from the maximum wavelength $\lambda_{max}$ to the minimum wavelength $\lambda_{min}$ is radiated to both the living body LT via the optical fiber 52a, the first coupler 53a, and the fiber bundle 60b and the collimating lens 56 via the optical fiber 52a, the first coupler 53a, and the fiber bundle 60d.

The light which enters the collimating lens 56 is converted to parallel-flux light and radiated to the reference mirror 25. This light is reflected by the reference mirror 25, the reflected light is made to pass through the collimating lens 56 again, and is radiated to the fiber bundle 60c as reference light. This reference light, incident on the fiber bundle 60c, is then radiated to the second coupler 53b.

Meanwhile, the light radiated into the living tissue LT travels inside the living tissue LT in the depth direction until reaching a portion associated with the wavelength of the light, and is reflected at the portion. The light reflected at the portion enters the fiber bundle 60a as object light.

In the second coupler 53b, the object light coming from the fiber bundle 60a interferes with the reference light coming from the fiber bundle 60c, thereby producing interference light. The interference light is radiated to the light detector 27 via the optical fiber 52d.

Then, the first interference signal provided from the light detector 27 is subjected to the same processing (the processing based on the above-mentioned equations (1) to (3)) as that performed in the biological observation apparatus 1 to obtain each beat frequency $f_b$. The values of the beat frequencies $f_b$ are stored in the memory 7b in order.

After the calculation of the beat frequency $f_b$ is completed, an ultrasound wave, which is a continuous wave, is radiated from the ultrasound transducer 26 to the living tissue LT, and then the light whose frequency is successively changed from the maximum wavelength $\lambda_{max}$ to the minimum wavelength $\lambda_{min}$ is radiated from the light source 21 to the living tissue LT through the path described above.

The light radiated into the living tissue LT is partly reflected in sequence at the respective ultrasound wave fronts $R_1, R_2, \cdots, R_N$ produced therein by the ultrasound wave, where the object light (reflected light) subjected to Doppler shift (frequency modulation) with frequencies $f_d$ is generated as described. This object light is made to enter the fiber bundle 60a and then radiated to the second coupler 53b serving as a light reception section.

In the second coupler 53b, the object light coming from the fiber bundle 60a interferes with the reference light coming from the fiber bundle 60c, thereby producing interference light. The interference light is radiated to the light detector 27 via the optical fiber 52d.

Then, the second interference signal provided from the light detector 27 is subjected to the same processing (the processing based on the above-mentioned equations (1) to (4)) as that performed in the biological observation apparatus 1 to obtain each beat frequency $f_{bd}$. The values of the beat frequencies $f_{bd}$ are stored in the memory 7b in order. Thereby, the scattering information of each portion, on which each of the wave fronts $R_1$, $R_2$, $\cdots$, $R_N$ of the ultrasound wave is generated, is uniquely identified based on the values of the beat frequency $f_b$ and the beat frequency $f_{bd}$ stored in the memory 7b. Hence, the biological observation apparatus 1A shown in Fig. 5 is able to have the advantages similar to those of the foregoing biological observation apparatus 1.

According to the biological observation apparatus and method of the present invention, information of a deeper portion of an object to be examined can be obtained with high S/N and quickly.

Although the description above contains many specificities, these should not be construed as limiting the scope of the invention but as merely providing illustrations of some of the presently preferred embodiments of the present invention. Thus the scope of the present invention should be determined by the appended claims.

**Claims**

1. A biological observation apparatus (1) comprising:

    a sound wave radiating unit (26) that radiates a sound wave into an object (LT) to be examined, the sound wave having a predetermined wavelength or frequency;
    a control unit (4) that controls a radiation state of the sound wave radiated from the sound wave radiating unit (26) to the object;
    a light radiating unit (21) that radiates light into the object (LT) while successively changing a wavelength of the light;
    a light receiving unit (22) that receives object light generated when the light radiated from the light radiating unit (21) is reflected at an area inside the object (LT); and
    a light detector (27) that obtains second interference light caused by interference between the radiated light and second object light received by the light receiving unit (22) when the sound wave is controlled to be radiated by the control unit (4);

    **characterized in that** the light detector (27) further obtains first interference light caused by interference between the radiated light and first object light received by the light receiving unit (22) when the sound wave is controlled not to be radiated by the control unit (4), and
    wherein the biological observation apparatus (1) further comprises a calculation unit (7) that calculates information of an area inside the object (LT) where the sound wave is radiated, based on the first interference light and the second interference light obtained by the light detector (27), the information being indicative of a scattered state of the radiated light.

2. The biological observation apparatus according to claim 1, wherein the calculation unit (7) detects the amount of frequency modulation produced between the first object light and the second object light depending on a variation with time of a wavelength or frequency of the light radiated from the light radiating unit (21), based on the first interference light and the second interference light, and calculates the information based on the amount of frequency modulation.

3. The biological observation apparatus according to claim 1, wherein the light radiating unit (21) radiates the light while successively decreasing the wavelength of the light.

4. The biological observation apparatus according to claim 1, wherein the light radiating unit (21) radiates the light while successively increasing the wavelength of the light.

5. The biological observation apparatus according to claim 1, wherein the sound wave radiated from the sound wave radiating unit (26) is a continuous wave.

6. The biological observation apparatus according to claim 1, wherein the sound wave radiated from the sound wave radiating unit (26) is a pulse wave.

7. The biological observation apparatus according to claim 1, wherein the sound wave radiated from the sound wave radiating unit (26) is an ultrasound wave.

8. The biological observation apparatus according to claim 1, wherein the sound wave is radiated along an axis of the light radiated from the light radiating unit (21).

9. The biological observation apparatus according to claim 1, further comprising a scan unit (3) that changes positions of the sound wave radiating unit (26) and the light radiating unit (21) with respect to the object (LT).

10. The biological observation apparatus according to claim 1, wherein the information includes an index of refraction inside the object (LT).

11. The biological observation apparatus according to claim 1, wherein the calculation unit (7) produces image data of the object (LT) based on the information.

12. The biological observation apparatus according to claim 11, further comprising a storage unit (7b) that stores the image data with the image data being related to a position inside the object (LT).

13. The biological observation apparatus according to claim 11, further comprising a display unit (8) that displays an image of the object (LT) based on the image data.

14. A biological observation method comprising:

radiating first light into an object (LT) to be examined while successively changing a wavelength of the first light;
receiving first object light which is generated when the first light is reflected at an area inside the object (LT);
radiating a sound wave into the object (LT), the sound wave having a predetermined wavelength or frequency;
radiating second light into the object (LT) while successively changing a wavelength of the second light;
receiving second object light which is generated when the second light is reflected at an area inside the object (LT); and
obtaining second interference light caused by interference between the second object light and the second light;

**characterized by** further comprising the steps of:

obtaining first interference light caused by interference between the first object light and the first light; and
calculating information of an area inside the object (LT) where the sound wave is radiated, based on the first interference light and the second interference light, the information being indicative of a scattered state of the second light.

15. The biological observation method according to claim 14, wherein the amount of frequency modulation produced between the first object light and the second object light depending on variations with time of wavelengths or frequencies of the first light and the second light is detected based on the first interference light and the second interference light, and the information is calculated based on the amount of frequency modulation.

16. The biological observation method according to claim 14, wherein the first light and the second light are radiated while the wavelengths of the first light and the second light are successively decreased.

17. The biological observation method according to claim 14, wherein the first light and the second light are radiated while the wavelengths of the first light and the second light are successively increased.

18. The biological observation method according to claim 14, wherein the sound wave is a continuous wave.

19. The biological observation method according to claim 14, wherein the sound wave is radiated along an axis of the first light and the second light.

**Patentansprüche**

1. Biologische Betrachtungsvorrichtung (1) mit:

einer Schallwellen-Abstrahleinheit (26), die eine Schallwelle in ein zu untersuchendes Objekt (LT) abstrahlt, wobei die Schallwelle eine vorbestimmte Wellenlänge oder Frequenz aufweist;

einer Steuereinheit (4), die einen Strahlungszustand der von der Schallwellen-Abstrahleinheit (26) auf das Objekt abgestrahlten Schallwelle steuert;

einer Licht-Abstrahleinheit (21), die Licht in das Objekt (LT) abstrahlt, während sie eine Wellenlänge des Lichts sukzessive ändert;

einer Licht-Empfangseinheit (22), die Objektlicht, das entsteht, wenn das von der Licht-Abstrahleinheit (21) abgestrahlte Licht an einem Bereich innerhalb des Objekts (LT) reflektiert wird, empfängt; und

einem Lichtdetektor (27), der zweites Interferenzlicht erhält, das durch Interferenz zwischen dem abgestrahlten Licht und von der Licht-Empfangseinheit (22) empfangenem zweitem Objektlicht entsteht, wenn die Schallwelle von der Steuereinheit (4) so gesteuert wird, dass sie abgestrahlt wird;

**dadurch gekennzeichnet, dass** der Lichtdetektor (27) ferner erstes Interferenzlicht erhält, das durch Interferenz zwischen dem abgestrahlten Licht und von der Licht-Empfangseinheit (22) empfangenem erstem Objektlicht entsteht, wenn die Schallwelle von der Steuereinheit (4) so gesteuert wird, dass sie nicht abgestrahlt wird, und wobei die biologische Betrachtungsvorrichtung (1) ferner eine Recheneinheit (7) umfasst, die Information von einem Bereich innerhalb des Objekts (LT), auf den die Schallwelle abgestrahlt wird, auf der Basis des von dem Lichtdetektor (27) erhaltenen ersten Interferenzlichts und zweiten Interferenzlichts berechnet, wobei die Information einen Streuungszustand des abgestrahlten Lichts angibt.

2. Biologische Betrachtungsvorrichtung nach Anspruch 1, wobei die Recheneinheit (7) den Betrag der Frequenzmodulation erfasst, die zwischen dem ersten Objektlicht und dem zweiten Objektlicht in Abhängigkeit von einer zeitlichen Schwankung einer Wellenlänge oder Frequenz des von der Licht-Abstrahleinheit (21) abgestrahlten Lichts erzeugt wird, basierend auf dem ersten Interferenzlicht und dem zweiten Interferenzlicht, und die Information auf der Basis des Frequenzmodulationsbetrags berechnet.

3. Biologische Betrachtungsvorrichtung nach Anspruch 1, wobei die Licht-Abstrahleinheit (21) das Licht abstrahlt, während sie die Wellenlänge des Lichts sukzessive verkürzt.

4. Biologische Betrachtungsvorrichtung nach Anspruch 1, wobei die Licht-Abstrahleinheit (21) das Licht abstrahlt, während sie die Wellenlänge des Lichts sukzessive verlängert.

5. Biologische Betrachtungsvorrichtung nach Anspruch 1, wobei die von der Schallwellen-Abstrahleinheit (26) abgestrahlte Schallwelle eine kontinuierliche Welle ist.

6. Biologische Betrachtungsvorrichtung nach Anspruch 1, wobei die von der Schallwellen-Abstrahleinheit (26) abgestrahlte Schallwelle eine Pulswelle ist.

7. Biologische Betrachtungsvorrichtung nach Anspruch 1, wobei die von der Schallwellen-Abstrahleinheit (26) abgestrahlte Schallwelle eine Ultraschallwelle ist.

8. Biologische Betrachtungsvorrichtung nach Anspruch 1, wobei die Schallwelle entlang einer Achse des von der Licht-Abstrahleinheit (21) abgestrahlten Lichts abgestrahlt wird.

9. Biologische Betrachtungsvorrichtung nach Anspruch 1, ferner mit einer Abtasteinheit (3), die Positionen der Schallwellen-Abstrahleinheit (26) und der Licht-Abstrahleinheit (21) in Bezug auf das Objekt (LT) ändert.

10. Biologische Betrachtungsvorrichtung nach Anspruch 1, wobei die Information einen Brechungsindex innerhalb des Objekts (LT) enthält.

11. Biologische Betrachtungsvorrichtung nach Anspruch 1, wobei die Recheneinheit (7) auf der Information basierende Bilddaten des Objekts (LT) erzeugt.

12. Biologische Betrachtungsvorrichtung nach Anspruch 11, ferner mit einer Speichereinheit (7b), welche die Bilddaten speichert, wobei sich die Bilddaten auf eine Position innerhalb des Objekts (LT) beziehen.

13. Biologische Betrachtungsvorrichtung nach Anspruch 11, ferner mit einer Anzeigeeinheit (8), die ein Bild des Objekts (LT) auf der Basis der Bilddaten anzeigt.

**14.** Biologisches Betrachtungsverfahren, umfassend:

Abstrahlen von erstem Licht in ein zu untersuchendes Objekt (LT), während eine Wellenlänge des ersten Lichts sukzessive geändert wird;

Empfangen ersten Objektlichts, das erzeugt wird, wenn das erste Licht an einem Bereich innerhalb des Objekts (LT) reflektiert wird;

Abstrahlen einer Schallwelle in das Objekt (LT), wobei die Schallwelle eine vorbestimmte Wellenlänge oder Frequenz hat;

Abstrahlen von zweitem Licht in das Objekt (LT), während eine Wellenlänge des zweiten Lichts sukzessive geändert wird;

Empfangen zweiten Objektlichts, das erzeugt wird, wenn das zweite Licht an einem Bereich innerhalb des Objekts (LT) reflektiert wird; und

Erhalten zweiten Interferenzlichts, das durch Interferenz zwischen dem zweiten Objektlicht und dem zweiten Licht entsteht;

**dadurch gekennzeichnet, dass** es ferner folgende Schritte umfasst:

Erhalten ersten Interferenzlichts, das durch Interferenz zwischen dem ersten Objektlicht und dem ersten Licht entsteht; und

Berechnen von Information aus einem Bereich innerhalb des Objekts (LT), auf den die Schallwelle abgestrahlt wird, basierend auf dem ersten Interferenzlicht und dem zweiten Interferenzlicht, wobei die Information einen Streuungszustand des zweiten Lichts angibt.

**15.** Biologisches Betrachtungsverfahren nach Anspruch 14, wobei der Betrag der Frequenzmodulation, die zwischen dem ersten Objektlicht und dem zweiten Objektlicht in Abhängigkeit von einer zeitlichen Schwankung von Wellenlängen oder Frequenzen des ersten Lichts und zweiten Lichts erzeugt wird, basierend auf dem ersten Interferenzlicht und dem zweiten Interferenzlicht erfasst wird, und die Information auf der Basis des Frequenzmodulationsbetrags berechnet wird.

**16.** Biologisches Betrachtungsverfahren nach Anspruch 14, wobei das erste Licht und das zweite Licht abgestrahlt werden, während die Wellenlängen des ersten Lichts und des zweiten Lichts sukzessive verkürzt werden.

**17.** Biologisches Betrachtungsverfahren nach Anspruch 14, wobei das erste Licht und das zweite Licht abgestrahlt werden, während die Wellenlängen des ersten Lichts und des zweiten Lichts sukzessive verlängert werden.

**18.** Biologisches Betrachtungsverfahren nach Anspruch 14, wobei die Schallwelle eine kontinuierliche Welle ist.

**19.** Biologisches Betrachtungsverfahren nach Anspruch 14, wobei die Schallwelle entlang einer Achse des ersten Lichts und des zweiten Lichts abgestrahlt wird.

**Revendications**

**1.** Appareil d'observation biologique (1) comprenant :

une unité de rayonnement d'onde sonore (26) qui fait rayonner une onde sonore dans un objet (LT) devant être examiné, l'onde sonore ayant une longueur d'onde ou une fréquence prédéterminée ;

une unité de commande (4) qui commande un état de rayonnement de l'onde sonore fait rayonner de l'unité de rayonnement d'onde sonore (26) jusqu'à l'objet ;

une unité de rayonnement de lumière (21) qui fait rayonner une lumière dans l'objet (LT) tout en changeant successivement une longueur d'onde de la lumière ;

une unité de réception de lumière (22) qui reçoit une lumière d'objet générée lorsque la lumière fait rayonner de l'unité de rayonnement de lumière (21) est réfléchie au niveau d'une zone à l'intérieur de l'objet (LT) ; et

un détecteur de lumière (27) qui obtient une deuxième lumière d'interférence causée par une interférence entre la lumière fait rayonner et une deuxième lumière d'objet reçue par l'unité de réception de lumière (22) lorsque l'onde sonore est commandée pour être fait rayonner par l'unité de commande (4) ;

**caractérisé en ce que** le détecteur de lumière (27) obtient en outre une première lumière d'interférence causée par une interférence entre la lumière fait rayonner et une première lumière d'objet reçue par l'unité de réception de lumière (22) lorsque l'onde sonore est commandée pour ne pas être fait rayonner par l'unité de commande (4), et dans lequel l'appareil d'observation biologique (1) comprend en outre une unité de calcul (7) qui calcule une information d'une zone à l'intérieur de l'objet (LT) où l'onde sonore est fait rayonner, sur la base de la première lumière d'interférence et de la deuxième lumière d'interférence obtenues par le détecteur de lumière (27), l'information étant indicatrice d'un état diffusé de la lumière fait rayonner.

2. Appareil d'observation biologique selon la revendication 1, dans lequel l'unité de calcul (7) détecte la quantité de modulation de fréquence produite entre la première lumière d'objet et la deuxième lumière d'objet en fonction d'une variation avec le temps d'une longueur d'onde ou d'une fréquence de la lumière fait rayonner à partir de l'unité de rayonnement de lumière (21), sur la base de la première lumière d'interférence et de la deuxième lumière d'interférence, et calcule l'information sur la base de la quantité de modulation de fréquence.

3. Appareil d'observation biologique selon la revendication 1, dans lequel l'unité de rayonnement de lumière (21) fait rayonner la lumière tout en diminuant successivement la longueur d'onde de la lumière.

4. Appareil d'observation biologique selon la revendication 1, dans lequel l'unité de rayonnement de lumière (21) fait rayonner la lumière tout en augmentant successivement la longueur d'onde de la lumière.

5. Appareil d'observation biologique selon la revendication 1, dans lequel l'onde sonore fait rayonner à partir de l'unité de rayonnement d'onde sonore (26) est une onde entretenue.

6. Appareil d'observation biologique selon la revendication 1, dans lequel l'onde sonore fait rayonner à partir de l'unité de rayonnement d'onde sonore (26) est une onde pulsée.

7. Appareil d'observation biologique selon la revendication 1, dans lequel l'onde sonore fait rayonner à partir de l'unité de rayonnement d'onde sonore (26) est une onde ultrasonore.

8. Appareil d'observation biologique selon la revendication 1, dans lequel l'onde sonore est fait rayonner le long d'un axe de la lumière fait rayonner à partir de l'unité de rayonnement de lumière (21).

9. Appareil d'observation biologique selon la revendication 1, comprenant en outre une unité de balayage (3) qui change des positions de l'unité de rayonnement d'onde sonore (26) et de l'unité de rayonnement de lumière (21) par rapport à l'objet (LT).

10. Appareil d'observation biologique selon la revendication 1, dans lequel l'information inclut un indice de réfraction à l'intérieur de l'objet (LT).

11. Appareil d'observation biologique selon la revendication 1, dans lequel l'unité de calcul (7) produit des données d'image de l'objet (LT) sur la base de l'information.

12. Appareil d'observation biologique selon la revendication 11, comprenant en outre une unité de stockage (7b) qui stocke les données d'image avec les données d'image se rapportant à une position à l'intérieur de l'objet (LT).

13. Appareil d'observation biologique selon la revendication 11, comprenant en outre une unité d'affichage (8) qui affiche une image de l'objet (LT) sur la base des données d'image.

14. Procédé d'observation biologique comprenant :

le rayonnement d'une première lumière dans un objet (LT) devant être examiné tout en changeant successivement une longueur d'onde de la première lumière ;
la réception d'une première lumière d'objet qui est générée lorsque la première lumière est réfléchie au niveau d'une zone à l'intérieur de l'objet (LT) ;
le rayonnement d'une onde sonore dans l'objet (LT), l'onde sonore ayant une longueur d'onde ou une fréquence prédéterminée ;
le rayonnement d'une deuxième lumière dans l'objet (LT) tout en changeant successivement une longueur d'onde de la deuxième lumière ;
la réception d'une deuxième lumière d'objet qui est générée lorsque la deuxième lumière est réfléchie au niveau d'une zone à l'intérieur de l'objet (LT) ; et

l'obtention d'une deuxième lumière d'interférence causée par une interférence entre la deuxième lumière d'objet et la deuxième lumière ;

**caractérisé en ce que** comprenant en outre les étapes de :

obtention d'une première lumière d'interférence causée par une interférence entre la première lumière d'objet et la première lumière ; et
calcul d'une information d'une zone à l'intérieur de l'objet (LT) où l'onde sonore est fait rayonner, sur la base de la première lumière d'interférence et de la deuxième lumière d'interférence, l'information étant indicatrice d'un état diffusé de la deuxième lumière.

15. Procédé d'observation biologique selon la revendication 14, dans lequel la quantité de modulation de fréquence produite entre la première lumière d'objet et la deuxième lumière d'objet en fonction de variations avec le temps de longueurs d'onde ou de fréquences de la première lumière et de la deuxième lumière est détectée sur la base de la première lumière d'interférence et de la deuxième lumière d'interférence, et l'information est calculée sur la base de la quantité de modulation de fréquence.

16. Procédé d'observation biologique selon la revendication 14, dans lequel la première lumière et la deuxième lumière sont fait rayonner tandis que les longueurs d'onde de la première lumière et de la deuxième lumière sont successivement diminuées.

17. Procédé d'observation biologique selon la revendication 14, dans lequel la première lumière et la deuxième lumière sont fait rayonner tandis que les longueurs d'onde de la première lumière et de la deuxième lumière sont successivement augmentées.

18. Procédé d'observation biologique selon la revendication 14, dans lequel l'onde sonore est une onde entretenue.

19. Procédé d'observation biologique selon la revendication 14, dans lequel l'onde sonore est fait rayonner le long d'un axe de la première lumière et de la deuxième lumière.

FIG.1

# FIG.2

START

EMIT LIGHT — S1

DETECT FIRST INTERFERENCE LIGHT — S2

CALCULATE BEAT FREQUENCY $f_b$ — S3

STORE BEAT FREQUENCY $f_b$ — S4

RADIATE ULTRASOUND WAVE — S5

EMIT LIGHT — S6

DETECT SECOND INTERFERENCE LIGHT — S7

CALCULATE BEAT FREQUENCY $f_{bd}$ — S8

STORE BEAT FREQUENCY $f_{bd}$ — S9

S12

CHANGE SCAN POSITION

CALCULATE DOPPLER SHIFT AMOUNT $f_d$ — S10

NO          SCAN END ?          S11 YES

END

# FIG.3

# FIG.4

FIG.5

EP 2 027 814 B1

## FIG.6

## FIG.7

20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 4419900
- US 2005256403 A
- WO 9850781 A

**Non-patent literature cited in the description**

- **T. YOSHIMURA ; O. MATOBA.** Biological Tomography Measurement Using Spectral-swept Interferometry. *OPTICAL AND ELECTRO-OPTICAL ENGINEERING CONTACT,* 2003, vol. 41 (7), 418-425
- **M. HISAKA ; T. SUGIURA ; S. KAWATA.** Optical Tomography of a Thick Scattering Medium Using Interaction between Pulsed Ultrasound and Light. *KOGAKU,* 2000, vol. 29, 631-634